# EUROPEAN PATENT APPLICATION

(11) **EP 3 824 800 A1**
(43) Date of publication of application: **26.05.2021**
(21) Application number: 19838203.8
(22) Date of filing: 16.07.2019
(51) Int. Cl.: A61B 5/00

(54) **ORAL SCANNER AND 3D OVERLAY IMAGE DISPLAY METHOD USING SAME**

(30) Priority: 17.07.2018 KR 20180082794
(71) Applicant: Ionebio Inc., Seongnam-si, Gyeonggi-do 13201 (KR); Acts Co., Ltd., Seoul 08592 (KR)
(72) Inventor: MOON, Hee Jong, Hanam-Si Gyeonggi-do 12914 (KR)
(74) Representative: Manitz Finsterwald Patent- und Rechtsanwaltspartnerschaft mbB
(86) International application number: PCT/KR2019/008742
(87) International publication number: WO 2020/017854

(57) **Abstract**

The present invention relates to an oral scanner which is inserted into a patient's mouth and scans the patient's mouth by means of non-contact manner to generate a three-dimensional model and a three-dimensional overlay image display method using the same. The oral scanner includes a projector which irradiates a source light source including a visible light source and an IR light source; a camera which acquires a three-dimensional image for a mouth surface and an IR image for an inside of the gingiva to sense reflected light for the source light source; and an image processor which combines the IR image and the three-dimensional image to generate a three-dimensional duplication model, wherein the IR image is irradiated in the mouth to be overlaid by the projector based on the three-dimensional duplication model. According to the oral scanner of the present invention, after generating a three-dimensional duplication model configured by a three-dimensional image for a surface in a mouth and an IR image for the inside of the gingiva, the IR image may be irradiated to be overlaid in the mouth in real time by referring to the three-dimensional duplication model so that the method may be useful for the dental treatment.

## Description

### [Technical Field]

The present invention relates to an oral scanner which is inserted into a patient's mouth to generate a three-dimensional model by means of non-contact scanning and a three-dimensional overlay image display method using the same.

### [Background Art]

Generating a duplication model which reproduces an appearance in the mouth is one of the most important processes in a dental care and is a process that requires both accuracy and efficiency to be satisfied. Most dental cares use a traditional impression taking method by making a plaster model to reproduce an appearance in the mouth.

Recently, as a digital technology is applied, a digitalized clinical method which replaces the existing method has been developed and applied in the dental care. As a part of this, an oral scanner which is inserted into a mouth of a patient to perform an impression-taking process by scanning the mouth in a contactless manner to acquire two-dimensional image data and processing the two-dimensional image data to generate a three-dimensional duplication model has been introduced.

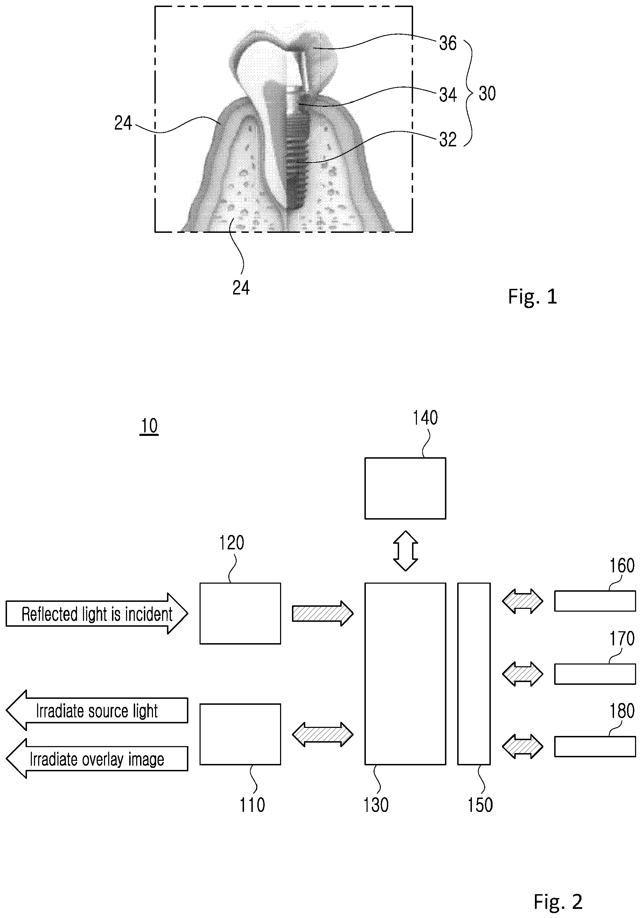

Such an oral scanner is being introduced and utilized in most oral cares such as impression taking and model or prostheses making, except for tooth scraping, and an application field thereof is expanding to an on-site treatment. In particular, among the dental cares, the implant procedure is one of the representative procedures for replacing a function of a missing tooth and as illustrated in FIG. 1, the implant 30 is configured by a fixture 32 fixed to the alveolar bone 24 deep in the gingiva 22, an abutment 34 coupled to an upper portion of the fixture 32, and a crown 36. The fixture 32 serves as an artificial tooth root and the abutment 34 is fastened with the fixture 32 through a bolt, stabilizes the gingiva 22 and prevent bacteria from penetrating into the alveolar bone 24 and the bone of the jaw before coupling the final crown 36.

In the meantime, in the on-site implant procedure, after a first procedure of implanting the fixture 32 into the alveolar bone 24, the incised gingiva 22 is sutured and then maintained for approximately three to six months to achieve synostosis of the fixture 32 to the alveolar bone 24, and then an incision process of the sutured gingiva 22 is performed again to expose the fixture 32 to be coupled to the abutment 34. In this case, when the gingiva 22 is excessively incised, problems such as the patient's pain, the risk of secondary infection, and the increase of the treatment period may be caused.

In order to solve the above-mentioned problem, it is necessary to suppress the degree of the incision of the gingiva 22 to the minimum after the first procedure. However, the oral scanner which has been known until now is limited to acquire only the three-dimensional duplication model for the mouth surface exposed to the outside, so that there is a limitation to be applied to the on-site implant procedure.

### [Disclosure]

### [Technical Problem]

In order to solve the above-described problems of the related art, an object of the present invention is to provide an oral scanner which has a small size to irradiate an image for the inside of the gingiva to be overlaid in the mouth and a three-dimensional overlay image display method using the same.

### [Technical Solution]

The present invention has been made to solve the above-described problems and the gist of the present invention will be described below as described in the claims.
(1) A oral scanner includes a projector which irradiates a source light source including a visible light source and an IR light source; a camera which acquires a three-dimensional image for a mouth surface and an IR image for an inside of the gingiva to sense reflected light for the source light source; and an image processor which combines the IR image and the three-dimensional image to generate a three-dimensional duplication model, and the IR image is irradiated in the mouth to be overlaid by the projector based on the three-dimensional duplication model.
(2) The oral scanner of (1) further includes a position sensing unit which senses an orientation of the oral scanner, and the image processor corrects the three-dimensional duplication model based on the orientation information received by the position sensing unit.
(3) In the oral scanner of (1), the projector includes a light source which includes a beam combiner and a visible light source and an IR light source disposed in the vicinity thereof; an illuminating unit which includes a display panel and a TIR prism; and a projective lens unit.
(4) In the oral scanner of (1), the camera includes a focal lens unit into which reflected light is incident; and a sensing unit which includes a beam splitter and a visual sensor and an IR sensor disposed in the vicinity thereof.
(5) A three-dimensional overlay image display method includes: (a) acquiring a three-dimensional image for a surface in the mouth by irradiating a visible light source as a pattern to sense reflected light thereof; (b) acquiring an IR image for the inside of the gingiva by irradiating an IR light source to sense reflected light thereof; (c) generating a three-dimensional duplication model by combining the three-dimensional image and the IR image; and (d) irradiating the IR image in the mouth to be overlaid by referring to the three-dimensional duplication model.
(6) In the 3D overlay image display method of (5), in the steps (a), (b), and (d), the light source is irradiated using the same irradiation optical system.
(7) In the 3D overlay image display method of (5), the irradiation optical system includes a light source which includes a beam combiner and a visible light source and an IR light source disposed in the vicinity thereof; an illuminating unit which includes a display panel and a TIR prism; and a projective lens unit.
(8) In the 3D overlay image display method of (5), in the steps (a) and (b), the reflected light is sensed using the same sensing optical system.
(9) In the 3D overlay image display method of (8), the sensing optical system includes a focal lens unit into which reflected light is incident; and a sensing unit which includes a beam splitter and a visual sensor and an IR sensor disposed in the vicinity thereof.
(10) In the 3D overlay image display method of (5), the step (d) is performed to be corrected according to the orientation of the three-dimensional duplication model.

### [Advantageous Effects]

According to the oral scanner of the present invention, after generating a three-dimensional duplication model configured by a three-dimensional image for a surface in the mouth and an IR image for the inside of the gingiva, the IR image may be irradiated to be overlaid in the mouth in real time by referring to the three-dimensional duplication model so that the method may be useful for the dental care. In particular, an implanting position of the fixture may be accurately confirmed based on the IR image which is irradiated to be overlaid in the mouth during the on-site implant procedure, so that the gingiva incision may be minimized only by simple drilling before assembling the abutment. Therefore, the pain of the patient is relieved during the procedure and the side effect of the procedure due to secondary contamination before and after the procedure may be minimized. Further, the oral scanner may function as both an irradiation optical system and a sensing optical system required to acquire the three-dimensional image and the IR image and also uses the irradiation optical system to irradiate the IR image to be overlaid so that it is advantageous for miniaturization of products.

### [Description of Drawings]

FIG. 1 is a cross-sectional view of the inside of the gingiva to which implant is performed.
FIG. 2 is a diagram of an oral scanner according to an embodiment of the present invention.
FIG. 3 is a flow chart of a three-dimensional overlay image display method using an oral scanner of FIG. 2.
FIG. 4 is a diagram of a projector which forms an irradiation optical system according to an embodiment of the present invention.
FIG. 5 is a diagram of a camera which forms a sensing optical system according to an embodiment of the present invention.
FIG. 6 is a view of an operation when a three-dimensional image for a mouth surface is acquired.
FIG. 7 is a view of an operation when an IR image for the inside of the gingiva is acquired.
FIG. 8 is a cross-sectional view of a skin penetration depth of IR ray according to a wavelength.
FIG. 9 is a view illustrating a state in which an IR image is irradiated to be overlaid in a mouth.

### [Best Mode]

Hereinafter, the present invention will be described in more detail with reference to exemplary embodiments. Prior to this, terms or words used in the present specification and claims should not be interpreted as being limited to typical or dictionary meanings, but should be interpreted as having meanings and concepts which comply with the technical spirit of the present invention, based on the principle that an inventor can appropriately define the concept of the term to describe his/her own invention in the best manner. Therefore, configurations illustrated in the embodiments described in the present specification are only the most preferred embodiment of the present invention and do not represent all of the technical spirit of the present invention, and thus it is to be understood that various equivalents and modified examples, which may replace the configurations, are possible when filing the present application. In the drawings, like components are denoted by like reference numerals. In addition, in the specification, unless explicitly described to the contrary, the word "comprise" and variations such as "comprises" or "comprising", will be understood to imply the inclusion of stated elements but not the exclusion of any other elements. In describing the present invention, when it is determined that the detailed description of the known art related to the present invention may obscure the gist of the present invention, the detailed description thereof will be omitted.

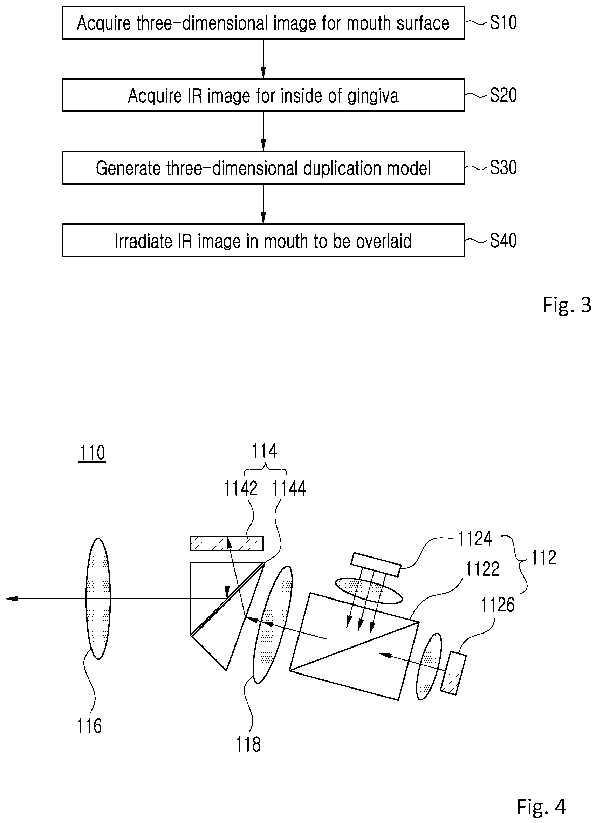

FIG. 2 illustrates a diagram of an oral scanner 10 according to an embodiment of the present invention. The oral scanner 10 includes a projector 110, a camera 120, and an image processor 130 as basic configurations and selectively further includes a position sensing unit 140. The oral scanner 10 may be connected to an external power device 160, storage device 170, or display device 180 through an interface 150. FIG. 3 illustrates a flow chart of a three-dimensional overlay image display method using the oral scanner 10 of FIG. 2. The three-dimensional overlay image display method corresponds to an operation of the oral scanner 10 and is performed by (a) a step S10 of acquiring a three-dimensional image for a surface in the mouth, (b) a step S20 of acquiring an IR image for an inside of a gingiva, (c) a step S30 of generating a three-dimensional duplication model, and (d) a step S40 of irradiating an IR image in the mouth to be overlaid and the step (d) may be selectively corrected according to an orientation of the three-dimensional duplication model using the position sensing unit 140.

Referring to FIGS. 2 and 3, the projector 110 of FIG. 2 configures an irradiation optical system and is related to the step (a) of FIG. 3, and desirably related to the step (d). The camera 120 of FIG. 2 configures the sensing optical system and is related to the step (b) of FIG. 3. The image processor 130 of FIG. 2 is related to the step (c) of FIG. 3. The position sensing unit 140 of FIG. 2 is related to the orientation correction of the oral scanner 10 in the step (d) of FIG. 3. According to the present disclosure, eventually, after combining the three-dimensional image (hereinafter, referred to as a "first image") for the surface in the mouth and the IR image (hereinafter, referred to as a "second image") for the inside of the gingiva to generate a three-dimensional duplication model, only the "second image" is irradiated by the projector 110 to be overlaid by referring to the three-dimensional duplication model, thereby facilitating the on-site implant procedure. Further, according to the present invention, even though different source light sources are used to acquire the "first image" and the "second image", the same irradiation optical system and sensing optical system are used and the irradiation optical system used to acquire the image is used to irradiate the overlay image so that the product size of the oral scanner is reduced.

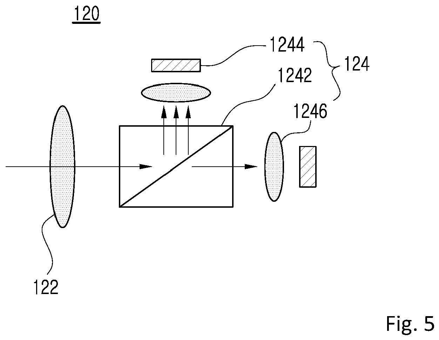

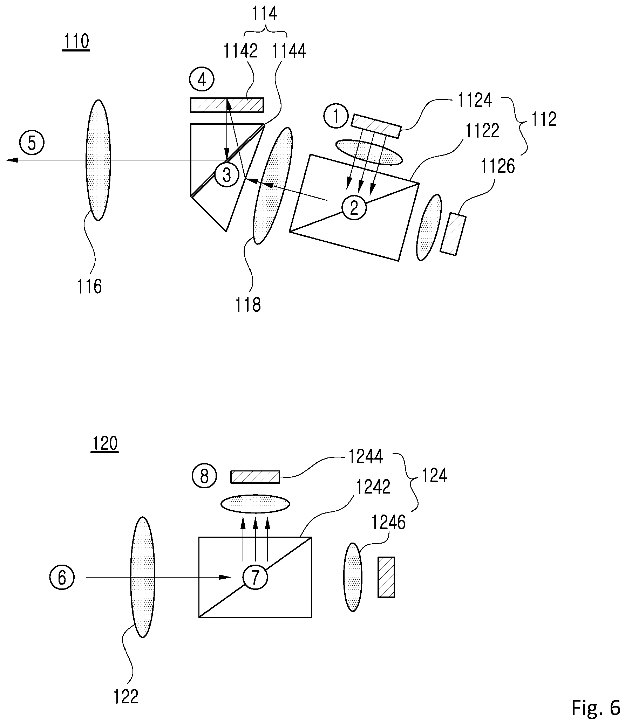

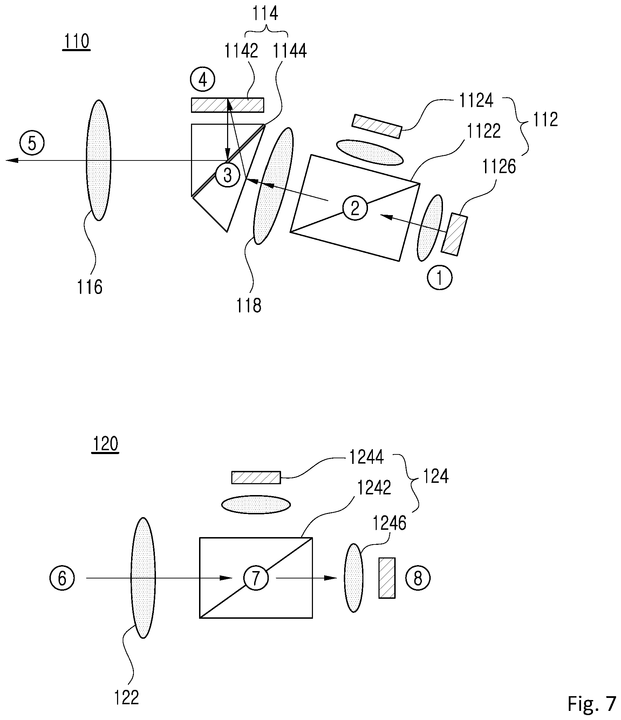

FIG. 4 illustrates a diagram of a projector 110 which forms an irradiation optical system according to an embodiment of the present invention and FIG. 5 illustrates a diagram of a camera 120 which forms a sensing optical system according to an embodiment of the present invention. FIGS. 6 and 7 illustrate processes of acquiring a three-dimensional image and an IR image for a mouth surface using the projector 110 and the camera 120. Here, each component of the oral scanner 10 will be sequentially described. In the following description, the irradiation optical system is understood as the same concept as the projector 110 and the sensing optical system is understood as the same concept as the camera 120.

Referring to FIG. 4, the projector 110 may perform both a basic function of irradiating a source light source to acquire the "first image" and the "second image" and a function of finally irradiating the "second image" into the mouth to be overlaid. The projector 110 includes a light source unit 112 including a beam combiner 1122, a visible light source 1124 and an IR light source 1126 disposed in the vicinity thereof, an illuminating unit 114 including a display panel 1142 and a TIR prism 1144, and a projective lens unit 116, and the visible light source 1124 and the IR light source 1126 of the light source unit 112 configure the source light source.

The visible light source 1124 basically serves as a source light source required to acquire the "first image". In this case, the visible light source 1124 is changed into a pattern type by the display panel 1142 of the illuminating unit 114 to be irradiated. The visible light source 1124 also serves as a source light source which irradiates the "second image" related to the position of an artifact in the gingiva, for example, an implanted position of a metal fixture at the time of the implant procedure onto a mouth surface to be overlaid to be identified with naked eyes. The visible light source 1124 may be configured by an LED light source and may be configured by white light or if necessary, configured by separate RGB light to extract a 3D image using a chromatic aberration according to each wavelength. An optical system with a large chromatic aberration has a focal surface which varies depending on RGB light, that is, has a projection length which varies depending on a wavelength, so that it is advantageous to extract 3D information.

The IR light source 1126 serves as a source light source required for acquiring the second image and in this case, a wavelength of the IR light source 1126 is desirably controlled to deeply penetrate into the hypodermis in the gingiva. The IR light source 1126 has a large chromatic aberration so that it is advantageous to extract a 3D image.

The beam combiner 1122 serves to combine the visible light source 1124 and the IR light source 1126 in the same optical axis in order to simultaneously or sequentially use the same optical system excluding a light source. The beam combiner 1122 may use a prism or have a plate shape which is subjected to a coating treatment. In this case, when the dichroic coating treatment is performed, the beam combiner is designed to reflect the visible light source 1124 and transmit the IR light source 1126.

The display panel 1142 of the illuminating unit 114 may be configured by DMD of Taxis Instruments Incorporated and a micro element, and serves to induce the change of an angle of light irradiated for every pixel according to image information to transmit the light to the projective lens unit 116. During this process, the TIR prism 1144 of the illuminating unit 114 has little angle change and serves to control the light to be suitable for a total reflection condition to separate incident light and emitted light in the same space.

In the meantime, a relay lens 118 interposed between the beam combiner 1122 and the illuminating unit 114 serves to induce light emitted from the beam combiner 1122 to be irradiated to the TIR prism 1144 of the illuminating unit 114 with a predetermined angle and illuminance.

The projective lens unit 116 serves to project image information generated by the display panel 1142 of the illuminating unit 114 into the mouth and generates a chromatic aberration to enhance the 3D effect.

Referring to FIG. 5, the camera 120 serves to sense reflected light for the source light source irradiated by the projector 110 to transmit the reflected light to the image processor 130. Specifically, the camera 120 includes a focal lens unit 122 into which the reflected light is incident and a sensing unit 124 including a beam splitter 1242 and a visual sensor 1244 and an IR sensor 1246 disposed in the vicinity thereof. The reflected light for the visible light source 1124 and the IR light source 1126 passes through the focal lens unit 122 to be incident into the sensing unit 124. The reflected light for the visible light source 1124 and the IR light source 1126 incident into the sensing unit 124 is split by the beam splitter 1242 and then sensed by the visual sensor 1244 and the IR sensor 1246 to acquire image information.

The image processor 130 generates 3D data for the "first image" and the "second image" and generates a three-dimensional duplication model based thereon. The three-dimensional duplication model is 3D data obtained by combining the "first image" and the "second image" and serves as a reference during the process of irradiating the "second image" in the mouth to be overlaid.

The position sensing unit 140 serves to sense an orientation of the oral scanner 10. The orientation information of the oral scanner 10 measured by the position sensing unit 140 is transmitted to the image processor 130 and the image processor 130 corrects the three-dimensional duplication model based on the orientation information. Since the overlay irradiation of the second image into the mouth is performed based on the three-dimensional duplication model which is corrected in real time according to the movement of the oral scanner 10, even though the oral scanner 10 moves during the procedure, the irradiation position of the second image which is overlaid in the mouth may be constantly maintained.

The position sensing unit 140 may be implemented by hardware or software means. As the hardware means, for example, an acceleration sensor, a geomagnetic sensor, or a GPS sensor is used to sense an XYZ coordinate position and αβγ angle information to sense orientation information of the oral scanner 10 or as the software means, for example, real-time image information which is sensed by the sensing optical system of the camera 120 is sensed to sense the orientation information of the oral scanner 10 by comparing the image information with image information which has been acquired in advance.

FIG. 6 illustrates an operational view of a first image acquiring process (S10 in FIG. 3) by the irradiation optical system of the projector 110 and the sensing optical system of the camera 120. Arabic numerals indicated in the drawings indicate paths through which visible light in the irradiation and reflection processes sequentially passes through each of the optical elements constituting the projector 110 and the camera 120. Referring to FIG. 6(a), first, according to the irradiation optical system of the projector 110, white light which is simultaneously irradiated from the visible light source 1124 or RGB light which is sequentially irradiated is reflected or transmitted by the beam combiner 1122 according to a wavelength and then illuminated onto the display panel 1142 by the TIR prism 1144 via the relay lens 118 and light which is changed into patterned light by the display panel 1142 is irradiated onto the surface in the mouth by the projective lens unit 116. Referring to FIG. 6(b), according to the sensing optical system of the camera 120, the RGB pattern light reflected from the mouth surface is introduced through the focal lens unit 122 and then is acquired to the visual sensor 1244 as the "first image" by the beam splitter 1242 to be transmitted to the image processor 130.

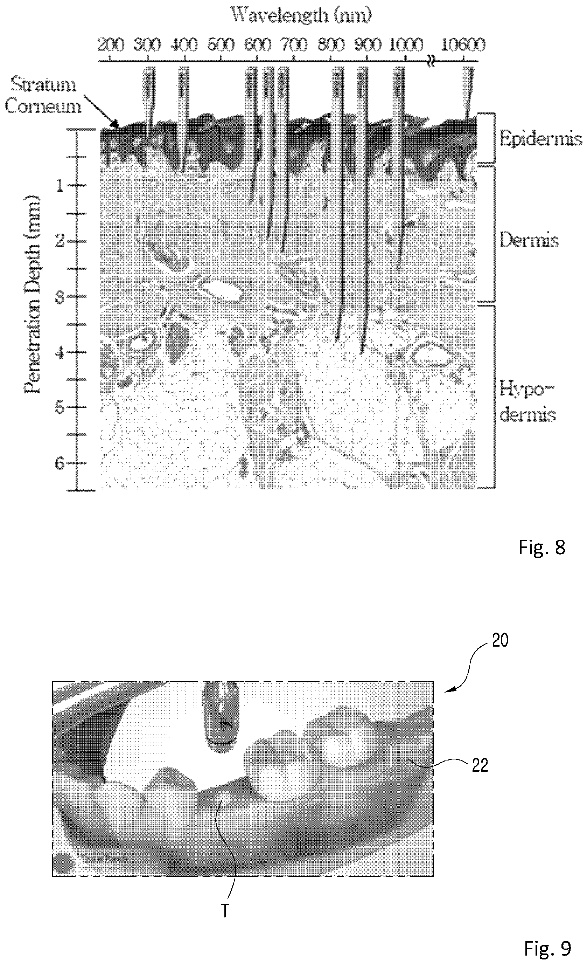

FIG. 7 illustrates an operational view of a "second image" acquiring process (S20 in FIG. 3) by the irradiation optical system of the projector 110 and the sensing optical system of the camera 120. Similarly to FIG. 6, arabic numerals indicated in the drawings indicate paths through which IR light in the irradiation and reflection processes sequentially passes through each of the optical elements constituting the projector 110 and the camera 120. Referring to FIG. 7(a), first, according to the irradiation optical system of the projector 110, IR light which is irradiated from the IR light source 1126 is reflected or transmitted by the beam combiner 1122 according to a wavelength and then illuminated onto the display panel 1142 by the TIR prism 1144 via the relay lens 118 and light which is changed into patterned light by the display panel 1142 is irradiated by the projective lens unit 116 to penetrate into the gingiva. Next, referring to FIG. 7(b), according to the sensing optical system of the camera 120, the IR pattern light reflected after penetrating into a skin is introduced through the focal lens unit 122 and then is acquired to the IR sensor 1246 as the second image by the beam splitter 1242 to be transmitted to the image processor 130. In this case, referring to FIG. 8 which represents a skin penetration depth of the IR light according to a wavelength, the wavelength of the IR light source 1126 is controlled in the range of 750 nm to 950 nm so that IR light may sufficiently penetrate into the vicinity of the hypodermis where an artifact such as a fixture of the implant is implanted.

In the meantime, after generating a three-dimensional duplication model (S30 of FIG. 3) by combining the "first image" of FIG. 6 and the "second image" of FIG. 7, the overlay irradiation process of the "second image" onto the surface in the mouth based on the three-dimensional duplication model (S40 of FIG. 3) is performed according to the operation of the irradiating optical system of the projector 110 as illustrated in FIG. 6(a). In this case, as the irradiated source light, the visible light source 1124 for displaying an implanting position of the fixture according to the "second image" is used.

FIG. 9 illustrates a shape in which an implanting position T of the fixture 32 according to the "second image" is irradiated to be overlaid in the mouth 20 using the oral scanner 10 according to the present invention. According to the oral scanner 10 of the present invention, after generating a three-dimensional duplication model configured by a three-dimensional image for a surface in the mouth 20 and an IR image for the inside of the gingiva 22, the IR image may be irradiated to be overlaid in the mouth 20 in real time by referring to the three-dimensional duplication model so that the method may be useful for the dental care. In particular, an implanting position T of the fixture may be accurately confirmed based on the IR image which is irradiated to be overlaid in the mouth during the on-site implant procedure, so that the incision of the gingiva 22 may be minimized only by simple drilling before assembling the abutment. Therefore, the pain of the patient is relieved during the procedure and the side effect of the procedure due to secondary contamination before and after the procedure may be minimized. Further, the oral scanner 10 may function as both an irradiation optical system and a sensing optical system required to acquire the three-dimensional image and the IR image and also use the irradiation optical system to irradiate the IR image to be overlaid so that it is advantageous for miniaturization of products.

The above description relates to specific exemplary embodiments of the present invention. The above-described exemplary embodiment according to the present invention is not understood to limit the matters disclosed for the purpose of explanation or the scope of the present invention, but it is understood that those skilled in the art can make various modifications or changes without departing from the gist of the present invention. Therefore, it can be understood that all changes and modifications corresponds to the scope of the invention disclosed in the claims or an equivalent thereof.

## Claims

1. An oral scanner, comprising:
a projector which irradiates a source light source including a visible light source and an IR light source;
a camera which acquires a three-dimensional image for a mouth surface and an IR image for an inside of the gingiva to sense reflected light for the source light source; and
an image processor which combines the IR image and the three-dimensional image to generate a three-dimensional duplication model,
wherein the IR image is irradiated in the mouth to be overlaid by the projector based on the three-dimensional duplication model.

2. The oral scanner according to claim 1, further comprising:
a position sensing unit which senses an orientation of the oral scanner,
wherein the image processor corrects the three-dimensional duplication model based on the orientation information received by the position sensing unit.

3. The oral scanner according to claim 1, wherein the projector includes:
a light source which includes a beam combiner and a visible light source and an IR light source disposed in the vicinity thereof;
an illuminating unit which includes a display panel and a TIR prism; and
a projective lens unit.

4. The oral scanner according to claim 1, wherein the camera includes:
a focal lens unit into which reflected light is incident; and
a sensing unit which includes a beam splitter and a visual sensor and an IR sensor disposed in the vicinity thereof.

5. A three-dimensional overlay image display method, comprising:
(a) acquiring a three-dimensional image for a surface in a mouth by irradiating a visible light source as a pattern to sense reflected light thereof;
(b) acquiring an IR image for the inside of the gingiva by irradiating an IR light source to sense reflected light thereof;
(c) generating a three-dimensional duplication model by combining the three-dimensional image and the IR image; and
(d) irradiating the IR image in the mouth to be overlaid by referring to the three-dimensional duplication model.

6. The 3D overlay image display method according to claim 5, wherein in the steps (a), (b), and (d), the light source is irradiated using the same irradiation optical system.

7. The 3D overlay image display method according to claim 6, wherein the irradiation optical system includes:
a light source which includes a beam combiner and a visible light source and an IR light source disposed in the vicinity thereof;
an illuminating unit which includes a display panel and a TIR prism; and
a projective lens unit.

8. The 3D overlay image display method according to claim 5, wherein in the steps (a) and (b), the reflected light is sensed using the same sensing optical system.

9. The 3D overlay image display method according to claim 8, wherein the sensing optical system includes:
a focal lens unit into which reflected light is incident; and
a sensing unit which includes a beam splitter and a visual sensor and an IR sensor disposed in the vicinity thereof.

10. The 3D overlay image display method according to claim 5, wherein the step (d) is performed to be corrected according to the orientation of the three-dimensional duplication model.
